(19) Europäisches Patentamt
European Patent Office
Office européen des brevets

(11) Veröffentlichungsnummer: **0 224 831**
**A2**

(12) # EUROPÄISCHE PATENTANMELDUNG

(21) Anmeldenummer: **86116246.9**

(22) Anmeldetag: **24.11.86**

(51) Int. Cl.⁴: **C07D 231/40** , C07D 231/38 , C07C 109/04 , A01N 43/56

(30) Priorität: **05.12.85 DE 3543033**
**28.05.86 DE 3617977**

(43) Veröffentlichungstag der Anmeldung:
**10.06.87 Patentblatt 87/24**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Gehring, Reinhold, Dr.**
**Dasnoeckel 49**
**D-5600 Wuppertal 11(DE)**
Erfinder: **Schallner, Otto, Dr.**
**Noldeweg 22**
**D-4019 Monheim(DE)**
Erfinder: **Stetter, Jörg, Dr.**
**Gellertweg 4**
**D-5600 Wuppertal 1(DE)**
Erfinder: **Marhold, Albrecht, Dr.**
**Carl-Duisberg-Strasse 329**
**D-5090 Leverkusen(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergische-Gladbach 2(DE)**
Erfinder: **Lürssen, Klaus, Dr.**
**August-Kierspel-Strasse 151**
**D-5060 Bergische-Gladbach 2(DE)**

(54) **5-Dichloracetamido-4-nitro-1-aryl-pyrazole.**

(57) Die Erfindung betrifft neue 5-Dichloracetamido-4-nitro-1-aryl-pyrazole der Formel (I),

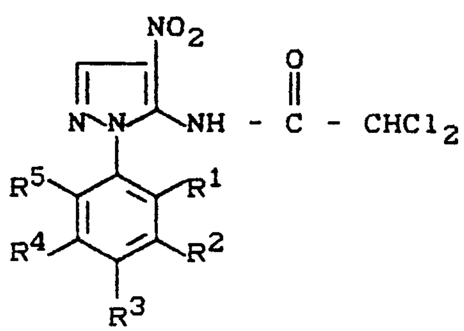

(I)

EP 0 224 831 A2

in welcher
$R^1$ für Wasserstoff, Fluor, Chlor oder Brom steht,
$R^2$ für Wasserstoff, Fluor oder Chlor steht,
$R^3$ für Halogen, Halogenalkyl, Halogenalkoxy, Halogenalkylthio oder Halogenalkylsulfonyl steht,
$R^4$ für Wasserstoff, Fluor oder Chlor steht und
$R^5$ für Fluor, Chlor oder Brom steht,
wobei jedoch
für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht, mehrere Verfahren zu ihrer Herstellung, ihre Verwendung als Herbizide und Wachstumsregulatoren sowie neue Zwischenprodukte zu deren Herstellung.

# 5-Dichloracetamido-4-nitro-1-aryl-pyrazole

Die Erfindung betrifft neue 5-Dichloracetamido-4-nitro-1-aryl-pyrazole, mehrere Verfahren zu ihrer Herstellung, ihre Verwendung als Herbizide und Wachstumsregulatoren sowie neue Zwischenprodukte zu deren Herstellung.

Es ist bereits bekannt, daß bestimmte 5-Halogenacylamino-4-nitro-1-aryl-pyrazole, wie beispielsweise das 5-(ω-Chlorbutyramido)-4-nitro-1-(2,6-dichlor-4-trifluormethylphenyl)-pyrazol herbizide Eigenschaften besitzen (vg. DE-OS 3 402 308).

Die herbizide Wirksamkeit dieser vorbekannten Verbindungen gegenüber Problemunkräutern ist jedoch, ebenso wie ihre Verträglichkeit gegenüber wichtigen Nutzpflanzen nicht immer in allen Anwendungsbereichen völlig zufriedenstellend.

Es wurden neue 5-Dichloracetamido-4-nitro-1-aryl-pyrazole der allgemeinen Formel (I),

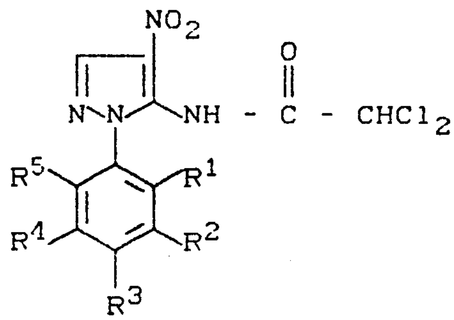

(I)

in welcher
$R^1$ für Wasserstoff, Fluor, Chlor oder Brom steht,
$R^2$ für Wasserstoff, Fluor oder Chlor steht,
$R^3$ für Halogen, Halogenalkyl, Halogenalkoxy, Halogenalkylthio oder Halogenalkylsulfonyl steht,
$R^4$ für Wasserstoff, Fluor oder Chlor steht und
$R^5$ für Fluor, Chlor oder Brom steht,
wobei jedoch
für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht,
gefunden.

Weiterhin wurde gefunden, daß man die neuen 5-Dichloracetamido-4-nitro-1-aryl-pyrazole der allgemeinen Formel (I),

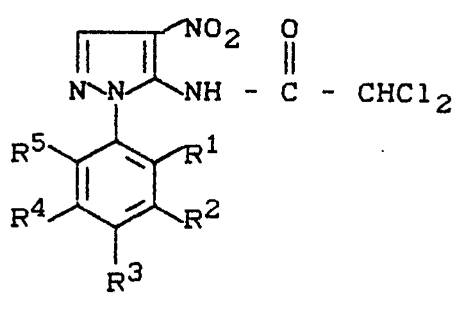

(I)

in welcher
$R^1$ für Wasserstoff, Fluor, Chlor oder Brom steht,
$R^2$ für Wasserstoff, Fluor oder Chlor steht,
$R^3$ für Halogen, Halogenalkyl, Halogenalkoxy, Halogenalkylthio oder Halogenalkylsulfonyl steht,
$R^4$ für Wasserstoff, Fluor oder Chlor steht und
$R^5$ für Fluor, Chlor oder Brom steht,

wobei jedoch
für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht,
erhält, wenn man

a) 5-Amino-4-nitro-1-aryl-pyrazole der Formel (II),

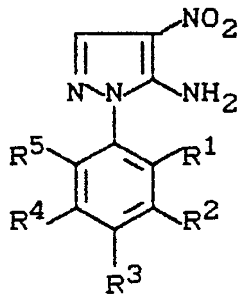

(II)

in welcher
$R^1$, $R^2$, $R^3$, $R^4$ und $R^5$ die oben angegebene Bedeutung haben
mit Dichloracetylverbindungen der Formel (III),

$Cl_2CH-\overset{O}{\overset{\|}{C}}-E$ (III)

in welcher
E für eine elektronenanziehende Abgangsgruppe steht,
gegebenenfalls in Gegenwart eines Verdünnungsmittels und gegebenenfalls in Gegenwart eines sauren oder basischen Katalysators umsetzt, oder
wenn man

b) in 4-Stellung unsubstituierte 5-Dichloracetamido-1-aryl-pyrazole der Formel (IV),

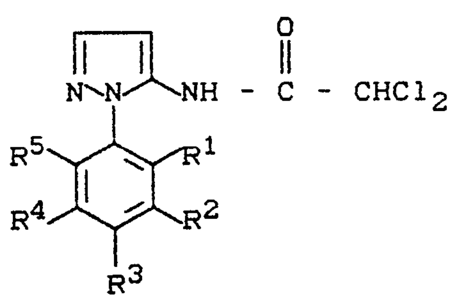

(IV)

in welcher
$R^1$, $R^2$, $R^3$, $R^4$ und $R^5$ die oben angegebene Bedeutung haben,
mit Salpetersäure gegebenenfalls in Gegenwart eines Verdünnungsmittels und gegebenenfalls in Gegenwart eines Katalysators umsetzt.

Schließlich wurde gefunden, daß die neuen 5-Dichloracetamido-4-nitro-1-aryl-pyrazole der Formel (I) herbizide und wachstumsregulierende Wirkung haben.

Überraschenderweise zeigen die erfindungsgemäßen 5-Dichlor-acetamido-4-nitro-1-aryl-pyrazole der allgemeinen Formel (I) eine erheblich bessere herbizide Wirksamkeit gegenüber Problemunkräutern bei vergleichbar guter Nutzpflanzenselektivität als die aus dem Stand der Technik bekannten 5-Halogenacylamido-4-nitro-1-aryl-pyrazole, wie beispielsweise das 5-(ω-Chlorbutyramido)-4-nitro-1-(2,6-dichlor-4-trifluormethyl-phenyl)-pyrazol, welche chemisch und wirkungsmäßig naheliegende Verbindungen sind.

Die erfindungsgemäßen 5-Dichloracetamido-4-nitro-1-aryl-pyrazole sind durch die Formel (I) allgemein definiert. Bevorzugt sind Verbindungen der Formel (I),
bei welchen
$R^1$ für Wasserstoff, Fluor, Chlor oder Brom steht,
$R^2$ für Wasserstoff, Fluor oder Chlor steht,

$R^3$ für Fluor, Chlor, Brom oder für jeweils geradkettiges oder verzweigtes halogenalkyl, Halogenalkoxy, Halogenalkylthio oder Halogenalkylsulfonyl mit jeweils 1 bis 4 Kohlenstoffatomen und 1 bis 9 gleichen oder verschiedenen Halogenatomen (insbesondere Fluor, Chlor oder Brom) steht,

$R^4$ für Wasserstoff, Fluor oder Chlor steht und

$R^5$ für Fluor, Chlor oder Brom steht,

wobei jedoch

für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht.

Besonders bevorzugt sind Verbindungen der Formel (I), bei welchen

$R^1$ für Wasserstoff, Fluor, Chlor oder Brom steht,

$R^2$ für Wasserstoff, Fluor oder Chlor steht,

$R^3$ für Fluor, Chlor, Brom, Trifluormethyl, Difluormethyl, Fluormethyl, Trichlormethyl, Dichlorfluormethyl, Difluorchlormethyl, Pentafluorethyl, Trifluormethoxy, Trichlormethoxy, Trifluormethylthio, Trifluormethylsulfonyl, Difluorchlormethylsulfonyl, Dichlorfluormethylsulfonyl oder für Pentafluorethylsulfonyl steht,

$R^4$ für Wasserstoff, Fluor oder Chlor steht und

$R^5$ für Fluor, Chlor oder Brom steht,

wobei jedoch

für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht.

Im einzelnen sei auf die bei den Herstellungsbeispielen genannten Verbindungen der Formel (I) verwiesen.

Verwendet man beispielsweise 5-Amino-4-nitro-1-(2,3,6-trichlor-4-trifluormethyl-phenyl)-pyrazol und Dichloracetanhydrid als Ausgangsstoffe, so läßt sich der Reaktionsablauf des erfindungsgemäßen Verfahrens (a) durch das folgende Formelschema darstellen:

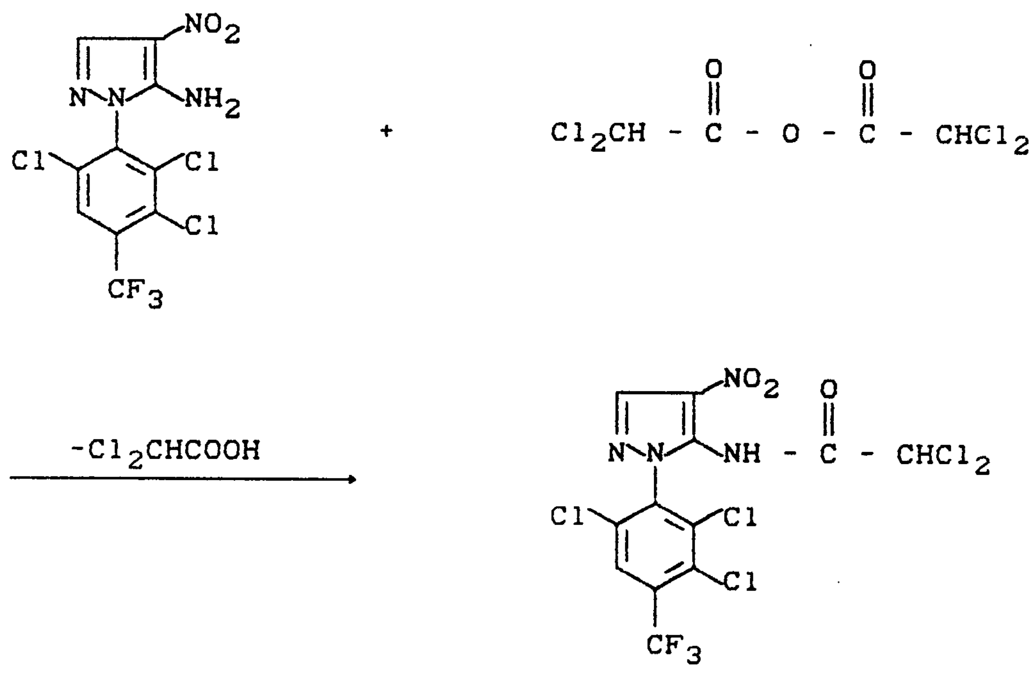


Verwendet man beispielsweise 5-Dichloracetamido-1-(2,6-dichlor-4-trifluormethylsulfonyl-phenyl)-pyrazol als Ausgangsverbindung, so läßt sich der Reaktionsablauf des erfindungsgemäßen Verfahrens (b) durch das folgende Formelschema darstellen:

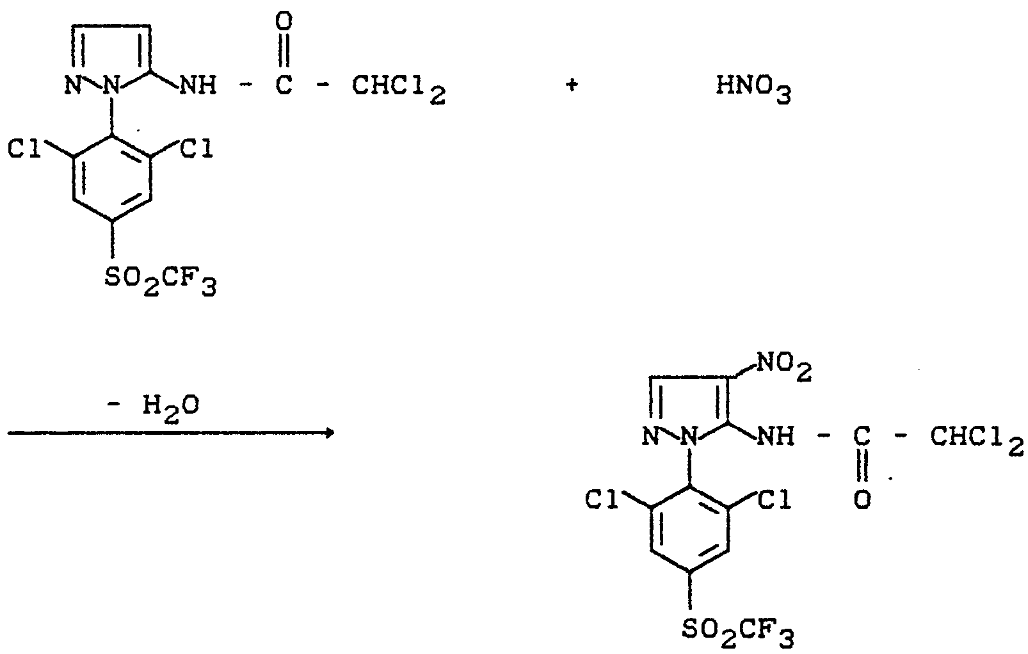


Die zur Durchführung des erfindungsgemäßen Vefahrens (a) als Ausgangsstoffe benötigten 5-Amino-4-nitro-pyrazole sind durch die Formel (II) allgemeinen definiert. In dieser Formel (II) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-4-nitro-pyrazole der Formel (II) sind teilweise bekannt (vgl. DE-OS 3 402 308) und lassen sich nach den dort beschriebenen Verfahren herstellen.

Noch nicht bekannt sind die 5-Amino-4-nitropyrazole der Formel (IIa),

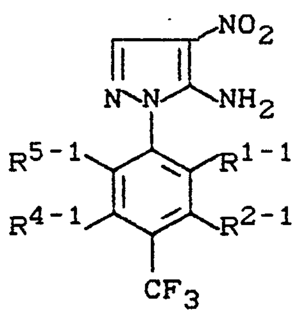

(IIa)

in welcher

$R^{1-1}$, $R^{2-1}$ und $R^{4-1}$ jeweils unabhängig voneinander für Wasserstoff, Fluor oder Chlor stehen und

$R^{5-1}$ für Fluor oder Chlor steht,

wobei jedoch die Kombinationen in denen

$R^{1-1}$, $R^{2-1}$, $R^{4-1}$ und $R^{5-1}$ gleichzeitig für Fluor stehen, $R^{1-1}$, $R^{2-1}$, $R^{4-1}$ und $R^{5-1}$ gleichzeitig für Chlor stehen, $R^{1-1}$ und $R^{5-1}$ für Chlor und $R^{2-1}$ und $R^{4-1}$ entweder gleichzeitig für Wasserstoff oder Fluor stehen, sowie $R^{1-1}$, $R^{2-1}$ und $R^{4-1}$ für Wasserstoff und $R^{5-1}$ gleichzeitig für Chlor stehen und $R^{1-1}$, $R^{2-1}$ und $R^{5-1}$ für Chlor und $R^{4-1}$ gleichzeitig für Wasserstoff stehen, ausgenommen sind.

Bevorzugt sind diejenigen Verbindungen der Formel (IIa), in denen $R^{1-1}$, $R^{2-1}$, $R^{4-1}$ und $R^{5-1}$ die bei der Beschreibung der Phenylhydrazine der Formel (V) angegebene bevorzugte Bedeutung haben.

Die Verbindungen der Formel (IIa) lassen sich herstellen, indem man Arylhydrazine der Formel (V)

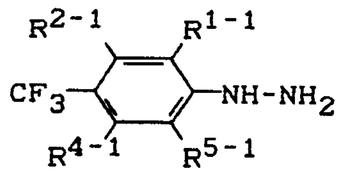

(V)

in welcher
$R^{1-1}$, $R^{2-1}$, $R^{4-1}$ und $R^{5-1}$ die oben angegebene Bedeutung haben,
mit 2-Halogenacrylnitrilen der Formel (VI),

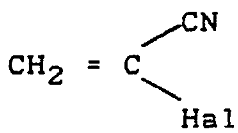

(VI)

in welcher
Hal für Halogen, insbesondere für Chlor oder Brom steht,
entweder zunächst in einer 1. Stufe, gegebenenfalls in Gegenwart eines Verdünnungsmittels, wie beispielsweise Eisessig oder Ethanol sowie gegebenenfalls in Gegenwart eines Reaktionshilfsmittels wie beispielsweise Natriumacetat bei Temperaturen zwischen -20 °C und +20 °C umsetzt zu den Arylhydrazin-Derivaten der Formel (VII),

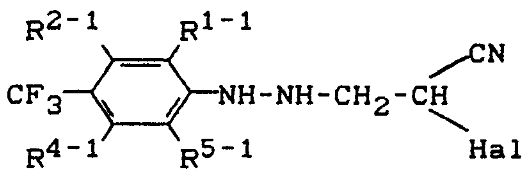

(VII)

in welcher
$R^{1-1}$, $R^{2-1}$, $R^{4-1}$ und $R^{5-1}$ und Hal die oben angegebene Bedeutung haben,
und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50 °C und +150 °C cyclisiert,
oder direkt in einem Reaktionsschritt ohne Isolierung der Zwischenstufe der Formel (VII) gegebenenfalls in Gegenwart eines Verdünnungsmittels wie beispielsweise Ethylenglykolmonoethylether oder Ethanol bei Temperaturen zwischen +50 °C und +150 °C cyclisiert und die so erhältlichen in 4-Stellung unsubstituierten 5-Amino-pyrazole der Formel (VIII),

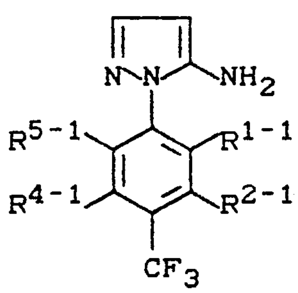

(VIII)

in welcher
$R^{1-1}$, $R^{2-1}$, $R^{4-1}$ und $R^{5-1}$ die oben angegebene Bedeutung haben,
in einer Folgereaktion mit einem Nitrierungsmittel wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen -20 °C und +50 °C

nitriert.

Dabei kann es gegebenenfalls von Vorteil sein, vor der Nitrierungsreaktion die Aminogruppe in der 5-Position des Pyrazolringes mit Hilfe üblicher Schutzgruppentechnik, beispielsweise durch Acylierung zu schützten und die Aminoschutzgruppe nach erfolgter Nitrierung ebenfalls in üblicher Art und Weise beispielsweise durch Verseifung mit wäßriger oder alkoholischer Base wieder abzuspalten.

Die neuen 5-Amino-4-nitropyrazole der Formel (II-a) zeigen in entsprechenden Konzentrationen auch eine sehr gute herbizide, insbesondere selektiv herbizide Wirksamkeit.

Die Arylhydrazine der Formel (V),

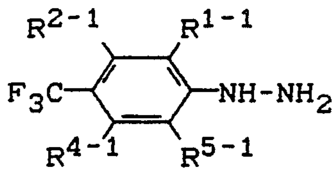

(V)

in welcher

$R^{1-1}$, $R^{2-1}$ und $R^{4-1}$ jeweils unabhängig voneinander für Wasserstoff, Fluor oder Chlor stehen und

$R^{5-1}$ für Fluor oder Chlor steht,

wobei jedoch die Kombinationen, in denen

$R^{1-1}$, $R^{2-1}$, $R^{4-1}$ und $R^{5-1}$ gleichzeitig für Fluor stehen, $R^{1-1}$, $R^{2-1}$, $R^{4-1}$ und $R^{5-1}$ gleichzeitig für Chlor stehen, $R^{1-1}$ und $R^{5-1}$ für Chlor und $R^{2-1}$ und $R^{4-1}$ entweder gleichzeitig für Wasserstoff oder Fluor stehen, sowie $R^{1-1}$, $R^{2-1}$ und $R^{4-1}$ für Wasserstoff und $R^{5-1}$ gleichzeitig für Chlor stehend und $R^{1-1}$, $R^{2-1}$ und $R^{5-1}$ für Chlor und $R^{4-1}$ gleichzeitig für Wasserstoff stehen, ausgenommen sind,

sind neu und Gegenstand der vorliegenden Erfindung.

Bevorzugt sind die folgenden Verbindungen der Formel (V):

| $R^{1-1}$ | $R^{2-1}$ | $R^{4-1}$ | $R^{5-1}$ |
|---|---|---|---|
| Cl | H | F | Cl |
| H | H | Cl | Cl |
| H | F | H | Cl |
| Cl | F | F | F |
| Cl | F | H | F |
| Cl | H | H | F |
| F | F | H | F |
| H | F | F | F |
| Cl | H | F | F |

Man erhält beispielsweise die neuen Arylhydrazine der Formel (V), wenn man Halogenaromaten der Formel (IX)

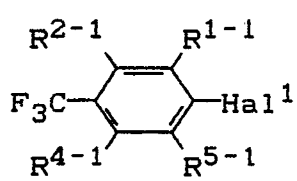

(IX)

in welcher

$R^{1-1}$, $R^{2-1}$, $R^{4-1}$ und $R^{5-1}$ die oben angegebene Bedeutung haben und
Hal' für Halogen, insbesondere für Fluor oder Chlor steht,
mit Hydrazin oder Hydrazinhydrat gegebenenfalls in Gegenwart eines Verdünnungsmittels umsetzt.

Die 2-Halogenacrylnitrile der Formel (VI) und die Halogenaromaten der Formel (IX) sind allgemein bekannte Verbindungen der organischen Chemie (vgl. z.B. EP-A-34 402 und US-P-4 388 472).

Die erfindungsgemäße Umsetzung zur Herstellung der Arylhydrazine der Formel (V) wird im allgemeinen in Gegenwart eines Lösungsmittels durchgeführt. Geeignete Lösungsmittel sind z.B. Alkohole, Ether, Etheralkohole, tertiäre Amine und Sulfone. Insbesondere geeignet sind Ethanol, Dioxan, Glykolmonomethylether, Triethylamin, Pyridin, Picolin und Tetramethylensulfon.

Geeignete Temperaturen für diese Umsetzung sind beispielsweise solche im Bereich von 0 bis 120°C, wobei insbesondere beim Einsatz von weniger reaktiven Verbindungen der Formel (IX) vorzugsweise Temperaturen im Bereich von 50 bis 120°C angewendet werden. Die Anwendung von Druck ist im allgemeinen nicht erforderlich, es sei denn, daß man die Umsetzung in einem Lösungsmittel durchführen möchte, das bei Normaldruck einen Siedepunkt hat, der unter der gewünschten Reaktionstemperatur liegt.

Im allgemeinen ist es vorteilhaft, wenn während der Reaktion nur geringe Überschüsse an Hydrazin vorliegen. Man legt deshalb vorzugsweise die Verbindung der Formel (IX), gegebenenfalls zusammen mit Lösungsmittel, vor und fügt dann Hydrazin dazu. Auch ist es im allgemeinen vorteilhaft den Einsatz von größeren Überschüssen von Hydrazin, z.B. von mehr als 1,2 Mol Hydrazin pro Mol der Verbindung der Formel (IX), zu vermeiden.

Sofern eine Verbindung der Formel (IX) mit Hal' = Chlor eingesetzt wird, ist es sehr vorteilhaft eine Base zuzusetzen, sofern nicht bereits als Lösungsmittel eine Base, z.B. Pyridin, verwendet wird. Geeignete Basen sind beispielsweise tertiäre Amine, wie Triethylamin, Pyridin und Picolin, sowie Carbonate, Hydrogencarbonate und Acetate von Alkalimetallen, wie Kaliumacetat, Natriumacetat, Kaliumcarbonat, Natriumhydrogencarbonat, Natriumcarbonat, Calciumhydroxyd und Calcium. Gegebenenfalls kann auch Hydrazin als diese Base dienen.

Das Hydrazin kann beispielsweise in der Form von Hydrazinhydrat eingesetzt werden, aber auch in wasserfreier Form oder bis zu 80 Gew.-% Wasser enthaltendem Hydrazin.

Im allgemeinen ist die Umsetzung nach 1 bis 15 Stunden beendet. Man kann dann das Reaktionsgemisch beispielsweise aufarbeiten, indem man, gegebenenfalls nach Abdestillieren von mindestens der Hälfte, vorzugsweise von mindestens 80 Gew.-% des vorhandenen Lösungsmittels (das wiederverwendet werden kann), das Reaktionsgemisch in kaltes Wasser einrührt, das kristallin anfallende Produkt der Formel (V) abtrennt, gegebenenfalls mit wenig Wasser nachwäscht und trocknet. Falls gewünscht, kann man das Produkt auch noch umkristallisieren, z.B. aus Cyclohexan oder Toluol.

Die Verbindungen der Formel (V) sind bei Raumtemperatur kristallin und können auf die beschriebene Weise mit guten Ausbeuten und in guten Reinheiten erhalten werden. Kleinere Anteile an Isomeren, die z.B. durch den Eintritt der $NH_2$-NH-Gruppe an anderer Stelle als in para-Position zur $CF_3$-Gruppe entstehen, werden durch die beschriebenen Aufarbeitung weitgehend entfernt.

Die zur Durchführung des erfindungsgemäßen Verfahrens (a) weiterhin als Ausgangsstoffe benötigten Dichloracetylverbindungen sind durch die Formel (III) allgemein definiert. In dieser Formel (III) steht E vorzugsweise für Halogen, insbesondere für Chlor oder Brom oder für einen Dichloracetyloxyrest.

Die Dichloracetylverbindungen der Formel (III) sind allgemein bekannte Verbindungen der organischen Chemie.

Die zur Durchführung des erfindungsgemäßen Verfahrens (b) als Ausgangsstoffe benötigten in 4-Stellung unsubstitu ierten 5-Dichloracetamido-1-aryl-pyrazole sind durch die Formel (IV) allgemein definiert. In dieser Formel (IV) stehen $R^1$, $R^2$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

Die in 4-Stellung unsubstituierten Dichloracetamido-1-aryl-pyrazole der Formel (IV) sind noch nicht bekannt. Man erhält sie jedoch in Analogie zu bekannten Verfahren (vgl. z.B. DE-OS 3 402 308), wenn man in 4-Stellung unsubstituierte 5-Amino-pyrazole der Formel (X),

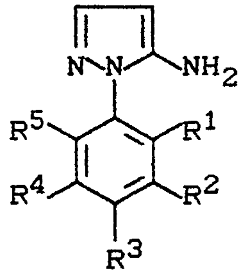

(X)

in welcher

$R^1$, $R^2$, $R^3$, $R^4$ und $R^5$ die oben angegebene Bedeutung haben,

mit Dichloracetylverbindungen der Formel (III),

$Cl_2CH - \overset{O}{\overset{\|}{C}} - E$ (III)

in welcher

E die oben angegebene Bedeutung hat,

gegebenenfalls in Gegenwart eines Verdünnungsmittels wie beispielsweise Dichlormethan und gegebenenfalls in Gegenwart eines Säurebindemittels wie beispielsweise Pyridin in Analogie zur Durchführung des erfindungsgemäßen Verfahrens (a) bei Temperaturen zwischen -20 °C und +120 °C acyliert.

Die in 4-Stellung unsubstituierten 4-Amino-pyrazole der Formel (X) sind teilweise bekannt (vgl. z.B. DE-OS 3 402 308) und teilweise neu (vgl. Verbindungen der allgemeinen Formel (VIII) und lassen sich in Analogie zu bekannten Verfahren herstellen.

Als Verdünnungsmittel zur Durchführung des erfindungsgemäßen Verfahrens (a) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Ketone wie Aceton oder Butanon, Nitrole, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren wie Essigsäure.

Das erfindungsgemäße Verfahren (a) wird gegebenenfalls in Gegenwart eines geeigneten sauren oder basischen Katalysators durchgeführt.

Als solche kommen alle üblichen anorganischen oder organischen Basen oder wasserfreie Protonensäuren in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU) oder wasserfreie Schwefelsäure, Phosphorsäure oder Salzsäure.

Die Reaktionstemperturen können bei der Durchführung des erfindungsgemäßen Verfahrens (a) in einem größeren Bereich variiert werden. Im allgemeinen arbeitet man bei Temperaturen zwischen -20 °C und +150 °C, vorzugsweise bei Temperaturen zwischen 0 °C und 120 °C.

Zur Durchführung des erfindungsgemäßen Verfahrens (a) setzt man pro Mol an 5-Amino-4-nitro-1-arylpyrazol der Formel (II) im allgemeinen 1.0 bis 5.0 Mol, vorzugsweise 1.0 bis 2.0 Mol an Dichloracetyl-Verbindung der Formel (III) und gegebenenfalls 1.0 bis 5.0 Mol, vorzugsweise 1.0 bis 2.0 Mol an Säurebindemittel ein. Die Reaktionsdurchführung, Aufarbeitung und Isolierung der Reaktionsprodukte der Formel (I) erfolgt nach üblichen, allgemein bekannten Verfahren.

Als Verdünnungsmittel zur Durchführung des erfindungsgemäßen Verfahrens (b) kommen alle üblicherweise für derartige Nitrierungsreaktionen verwendbaren Lösungsmittel in Frage. Vorzugsweise verwendet man die als Reagenzien in Frage kommenden Säuren oder deren Gemische mit Katalysatorsäure, wie beispielsweise Schwefelsäure, Salpetersäure, Acetanhydrid oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel in Frage.

Als Katalysatoren oder Reaktionshilfsmittel zur Durchführung des erfindungsgemäßen Verfahrens (b) kommen ebenfalls die für derartige Nitrierungen üblichen Katalysatoren in Frage; vorzugsweise verwendet man saure Katalysatoren, wie beispielsweise Schwefelsäure oder Acetanhyrid.

Die Reaktionstemperaturen können bei der Durchführung des erfindungsgemäßen Verfahrens (b) in einem größeren Bereich variiert werden. Im allgemeinen arbeitet man bei Temperaturen zwischen -50 °C und +120 °C, vorzugsweise bei Temperaturen zwischen -20 °C und +150 °C.

Zur Durchführung des erfindungsgemäßen Verfahrens (b) setzt man pro Mol an in 4-Stellung unsubstituierten 5-Dichloracetamido-1-aryl-pyrazol der Formel (IV) im allgemeinen 1.0 bis 100.0 Mol, vorzugsweise 1.0 bis 50.0 Mol an Salpetersäure und gegebenenfalls 0.1 bis 10 Mol an Katalysator ein. Die Reaktionsdurchführung, Aufarbeitung und Isolierung der Reaktionsprodukte der Formel (I) erfolgt nach üblichen, allgemeinen bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Cenaurea.

Diktoyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono-und dikotyler Unkräuter insbesondere in monokotylen Kulturen wie Gerste oder Weizen einsetzen.

Auch die Vorprodukte der Formel (IV) zeigen in entsprechenden Aufwandmengen eine gute herbizide Wirksamkeit.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Schäume, wirkstoffimprägnierte Natur-und synthetische Stoffe sowie Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chorethyllene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigen gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktio nierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglycol-Ether, Alkylsulfonate, Alkylsulfate, Arylsulfonate sowie Eiweißhydrolysate; als Dispergiermitel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-Chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; 2-[4-(3,5-Dichlorpyrid-2-yloxy)-phenoxy]-propionsäure-(2-benzyloxyethylester); --(trimethylsilylmethylester) oder -(2,2-diethoxyethylester); Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; N-Methyl-2-(1,3-benzthiazol-2-yloxy)-acetanilid; N,N-Diisopropyl-S-(2,3,3-trichlorallyl)-thiolcarbamat; N-(1-Ethylpropyl)-3,4-dimethyl-2,6-dinitroanilin; 3,5-Dibrom-4-hydroxy-benzonitril; 2-[4-(2,4-Dichlorphenoxy)-phenoxy]-propionsäure-methylester sind gegebenenfalls von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5,0 kg pro ha.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele:

Beispiel 1:

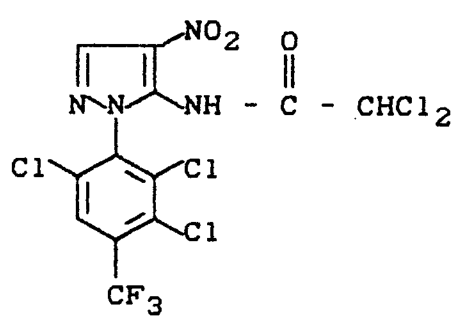

(Verfahren (b))

Zu 5 g (0.0113 Mol) 5-Dichloracetamido-1-(2,3,6-trichlor-4-trifluormethyl-phenyl)-pyrazol in 10 ml Eisessig gibt man bei Raumtemperatur nacheinander 1,2 ml (0.0129 Mol) Acetanhydrid und 0,6 ml (0.0143 Mol) 98-prozentige Salpetersäure. Nach 20-stündigem Rühren wird die Mischung im Vakuum eingeengt, der Rückstand in 50 ml Dichlormethan aufgenommen und nacheinander mit gesättigter Natriumhydrogencarbonat-und gesättigter Kochsalzlösung gewaschen. Die organische Phase wird über Magnesiumsulfat getrocknet und im Vakuum vom Lösungsmittel befreit. Man erhält 4,2 g (76,4 % der Theorie) an 5-Dichloracetamido-4-nitro-1-(2,3,6-trichlor-4-trifluormethyl-phenyl)-pyrazol vom Schmelzpunkt 112 °C-120 °C.

Beispiel 2:

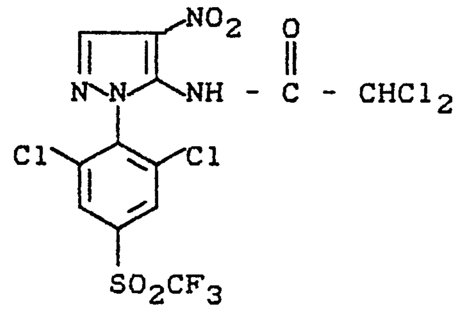

Verfahren (b)

Zu 5,2 g (0.0110 Mol) 5-Dichloracetamido-1-(2,6-dichlor-4-trifluormethylsulfonyl)-pyrazol in 10 ml Eisessig gibt man bei Raumtemperatur nacheinander 1,2 ml (0.0129 Mol) Acetanhydrid und 0,6 ml (0.0143 Mol) 98-prozentige Salpetersäure. Nach 20-stündigem Rühren wird die Mischung im Vakuum eingeengt, der Rückstand in 50 ml Dichlormethan aufgenommen und nacheinander mit gesättigter Natriumhydrogencarbonat-und gesättigter Kochsalzlösung gewaschen. Die organische Phase wird über Magnesiumsulfat getrocknet und im Vakuum vom Lösungsmittel befreit. Man erhält 4,7 g (83,9 % der Theorie) an 5-Dichloracetamido-4-nitro-1-(2,6-dichlor-4-trifluormethylsulfonyl-phenyl)-pyrazol vom Schmelzpunkt 138 °C-142 °C.

<u>Beispiel 3:</u>

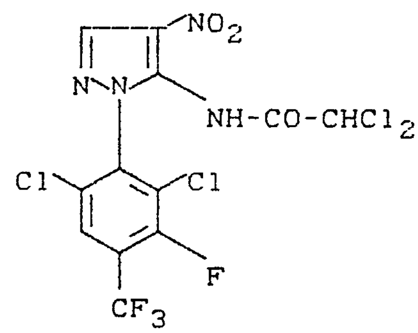

Verfahren (a)

Zu 3,0 g (0,0084 Mol) 5-Amino-1-(2,6-dichlor-3-fluor-4-trifluormethylphenyl)-4-nitropyrazol in 10 ml Dichloressigsäure gibt man bei Raumtemperatur 3,0 ml (0,031 Mol) Dichloracetylchlorid und 2 Tropfen 96 %ige Schwefelsäure. Nach 4-stündigem Rühren bei 140° C wird die Mischung auf 50 ml Wasser und 50 ml Dichlormethan ausgetragen. Die organische Phase wird abgetrennt, nacheinander mit gesättigter Natriumhydrogencarbonat-und gesättigter Natriumchloridlösung gewaschen, über Magnesiumsulfat getrocknet und im Vakuum vom Lösungsmittel befreit. Man erhält 3,2 g (81,5 % der Theorie) an 5-Dichloracetamido-(2,6-dichlor-3-fluor-4-trifluormethylphenyl)-4-nitropyrazol vom Schmelzpunkt 115-117°C.

In entsprechender Weise und gemäß den allgemeinen Angaben zur Herstellung erhält man die folgenden 5-Dichloracetamido-4-nitro-1-aryl-pyrazole der allgemeinen Formel (I):

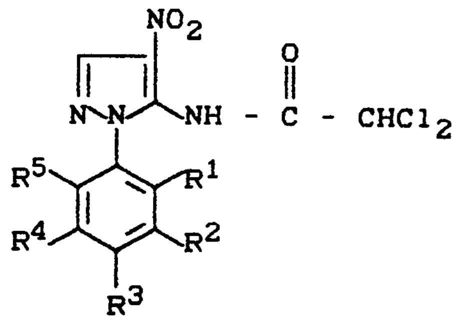

(I)

Tabelle 1

| Bsp.-Nr. | 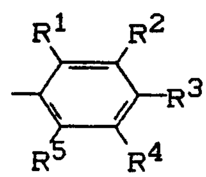 | Schmelzpunkt/° C |
|---|---|---|
| 4 | 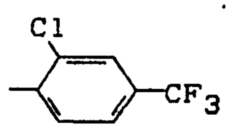 | 134-138 |
| 5 | 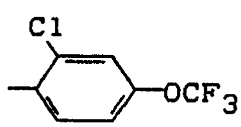 | 82-85 |
| 6 | 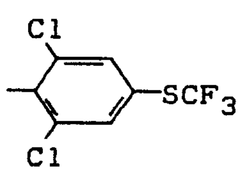 | 118-119 |

<u>Tabelle 1</u> (Fortsetzung)

| Bsp.-Nr. | 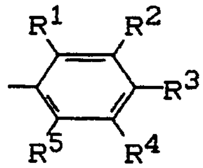 | Schmelzpunkt/° C |
| --- | --- | --- |
| 7 | 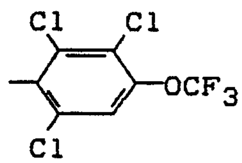 | 88 |
| 8 | 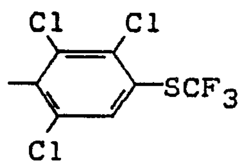 | 106 |
| 9 | 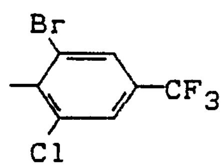 | 136 |
| 10 | 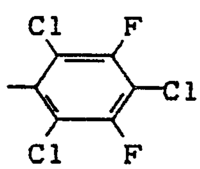 | 160-164 |
| 11 | 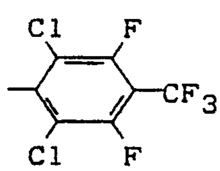 | 120-121 |
| 12 | 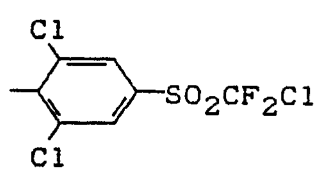 | 134 |

Tabelle 1 (Fortsetzung)

| Bsp.-Nr. | 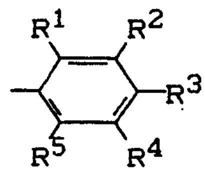 | Schmelzpunkt/°C |
|---|---|---|
| 13 | 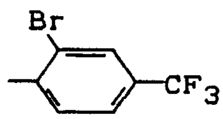 | 143 |
| 14 | 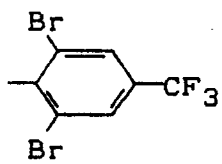 | |
| 15 | 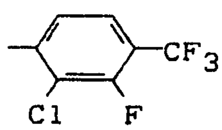 | 141-148 |
| 16 | 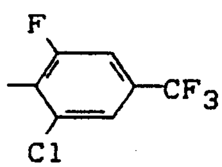 | 101-104 |
| 17 | 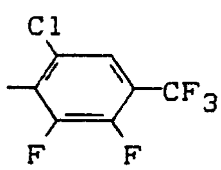 | |
| 18 | 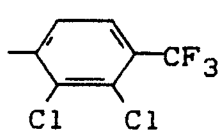 | |

Tabelle 1 (Fortsetzung)

| Bsp.-Nr. | 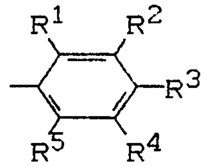 | Schmelzpunkt/° C |
|---|---|---|
| 19 | 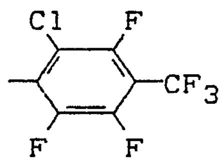 | |
| 20 | 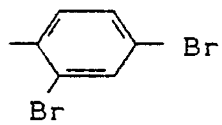 | 55 - 65 |

Herstellung der Ausgangsprodukte der Formel (IIa)

Beispiel IIa-1

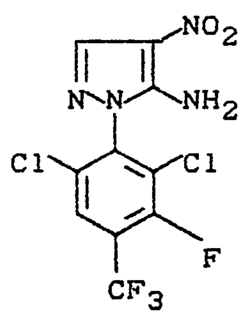

In eine siedende Lösung von 2,3 ml (0,034 Mol) 67-prozentiger Salpetersäure und 0,1 g Harnstoff in 30 ml Wasser trägt man 10 g (0,032 Mol) 5-Amino-1-(2,6-dichlor-3-fluor-4-trifluormethylphenyl)-pyrazol portionsweise ein. Man läßt die Suspension erkalten, filtriert den Niederschlag ab und trocknet ihn im Vakuum bei Raumtemperatur. Der trockene Niederschlag wird bei -5°C in 40 ml 98-prozentige Schwefelsäure eingetragen und 12 Stunden bei 0-5°C gerührt. Danach trägt man die Reaktionsmischung auf 350 ml Wasser aus, kocht kurz auf, läßt auf Raumtemperatur abkühlen und filtriet den Niederschlag ab. Der Niederschlag wird mit Wasser neutral gewaschen und im Vakuum bei 40-50°C getrocknet. Man erhält 7,0 g (61 % der Theorie) an 5-Amino-1-(2,6-dichlor-3-fluor-4-trifluormethylphenyl)-4-nitropyrazol vom Schmelzpunkt 156°C.

In entsprechender Weise und gemäß den allgemeinen Angaben zur Herstellung erhält man die folgenden 5-Amino-1-aryl-4-nitropyrazole der allgemeinen Formel (IIa)

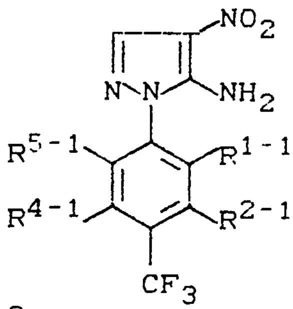

(IIa)

Tabelle 2

| Bsp.-Nr. | 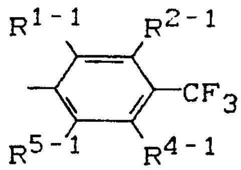 | Schmelzpunkt/°C |
|---|---|---|
| IIa-2 | 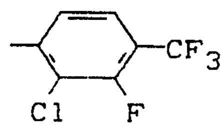 | 125-128 |
| IIa-3 | 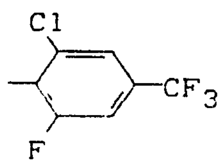 | 171 |
| IIa-4 | 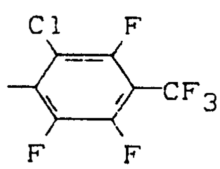 | 112 |
| IIa-5 | 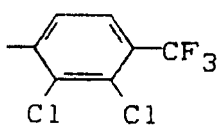 | |
| IIa-6 | 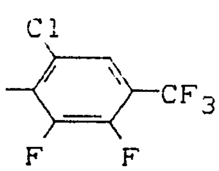 | |
| IIa-7 | 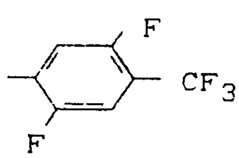 | 96-99 |

<u>Herstellung der Ausgangsprodukte der Formel (IV):</u>

<u>Beispiel IV-1:</u>

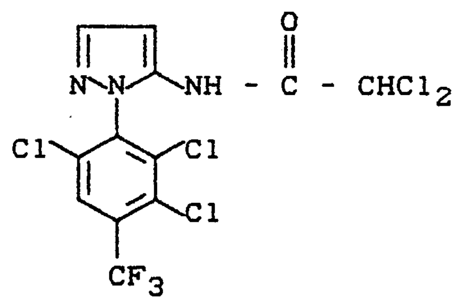

Zu 8,0 g (0.024 Mol) 5-Amino-1-(2,3,6-trichlor-4-trifluormethyl-phenyl)-pyrazol in 80 ml Dichlormethan gibt man nacheinander bei 5 °C bis 10 °C 15,3 g (0.145 Mol) wasserfreies Natriumcarbonat und 4,76 ml - (0.048 Mol) 98-prozentiges Dichloracetylchlorid. Es wird 4 Stunden bei 5 °C-10 °C und 13 Stunden bei Raumtemperatur gerührt. Die Mischung wird mit 80 ml Dichlormethan verdünnt, filtriert und nacheinander mit Wasser und gesättigter Kochsalzlösung gewaschen. Die organische Phase wird über Magnesiumsulfat getrocknet und im Vakuum vom Lösungsmittel befreit. Man erhält 10,0 g (93,5 % der Theorie) an 5-Dichloracetamido-1-(2,3,6-trichlor-4-trifluormethyl-phenyl)-pyrazol vom Schmelzpunkt 156 °C-161 °C.

In entsprechender Weise und gemäß den allgemeinen Angaben zur Herstellung erhält man die folgenden in 4-Stellung unsubstituierten 5-Dichloracetamido-1-aryl-pyrazole der allgemeinen Formel (IV):

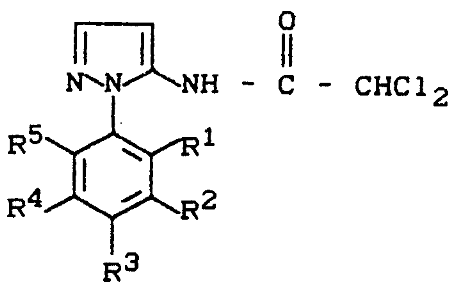 (IV)

<u>Tabelle 3</u>

| Bsp.-Nr. | 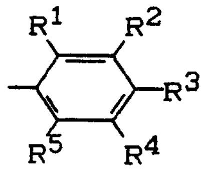 | Schmelzpunkt/° C |
|---|---|---|
| IV-2 | 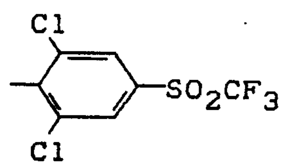 | 69-73 |
| IV-3 | 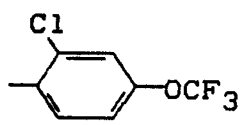 | 94-97 |
| IV-4 | 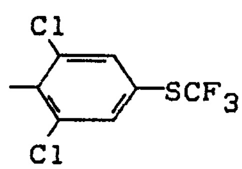 | 150-151 |

Tabelle 3 (Fortsetzung)

| Bsp.-Nr. | 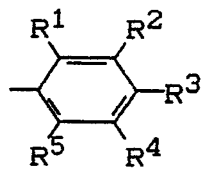 | Schmelzpunkt/° C |
|---|---|---|
| IV-5 | 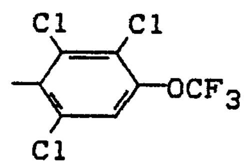 | 138 |
| IV-6 | 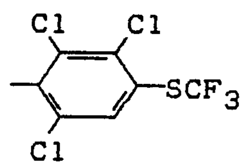 | 146 |
| IV-7 | 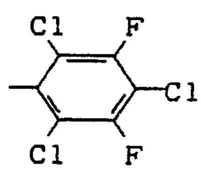 | 128-129 |
| IV-8 | 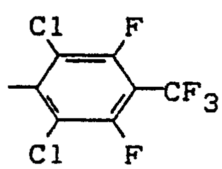 | 146-149 |
| IV-9 | 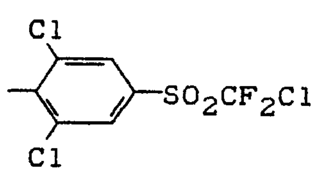 | 146-148 |
| IV-10 | 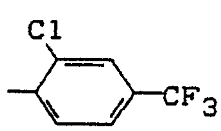 | 112-114 |

Tabelle 3 (Fortsetzung)

| Bsp.-Nr. | 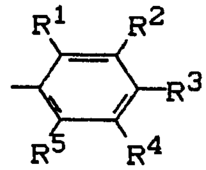 | Schmelzpunkt/° C |
|---|---|---|
| IV-11 | 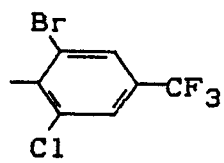 | 156 |
| IV-12 | 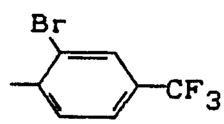 | |
| IV-13 | 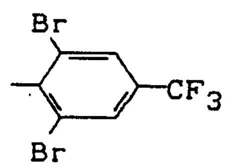 | |
| IV-14 | 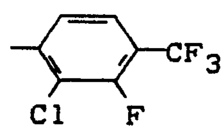 | 131-134 |
| IV-15 | 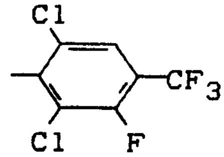 | 162-165 |
| IV-16 | 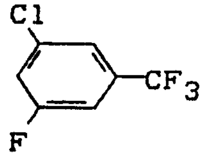 | 118-121 |

Tabelle 3 (Fortsetzung)

| Bsp.-Nr. | 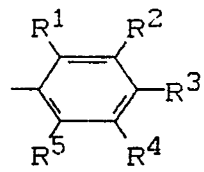 | Schmelzpunkt/°C |
|---|---|---|
| IV-17 | 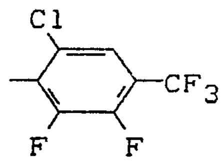 | |
| IV-18 | 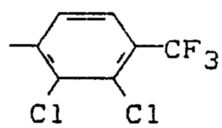 | |
| IV-19 | 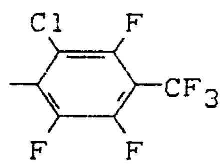 | 112-116 |

Herstellung der Ausgangsprodukte der Formel (V)

Beispiel V-1

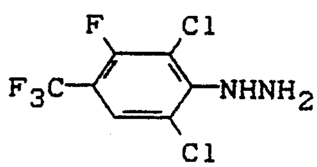

In 1000 ml Ethanol werden 470 g 3,5-Dichlor-2,4-difluorbenzotrifluorid vorgelegt und 142 g Hydrazinhydrat zudosiert und anschließend für 3 Stunden zum Rückfluß erhitzt. Danach wird das Lösungsmittel unter reduziertem Druck abdestilliert und der Rückstand in 1 l kaltes Wasser eingerührt. Nach 30 Minuten wird abgesaugt und das Festprodukt im Umluftschrank getrocknet. Man erhält 445 g 2,6-Dichlor-3-fluor-4-trifluormethyl-phenylhydrazin mit einem Schmelzpunkt von 50 bis 51 °C.

Beispiel V-2

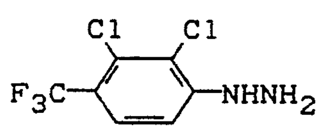

Es werden 100 g 2,3,4-Trichlorbenzotrifluorid vorgelegt, 200 ml Pyridin zugefügt, anschließend 100 g Hydrazinhydrat und dann 12 Stunden auf Rückflußtemperatur erhitzt. Danach wird das Pyridin zu 90 % abdestilliert und der verbleibende Rückstand in 250 ml Wasser eingerührt. Das kristalline Produkt wird abgesaugt, mit etwas Wasser gewaschen und getrocknet. Man erhält 78 g 2,3-Dichlor-4-trifluormethyl-phenylhydrazin mit einem Schmelzpunkt von 79 bis 80°C.

In entsprechender Weise, insbesondere analog Beispiel V-1 und gemäß den allgemeinen Angaben zur Herstellung erhält man die in der folgenden Tabelle aufgeführten Verbindungen der Formel (V):

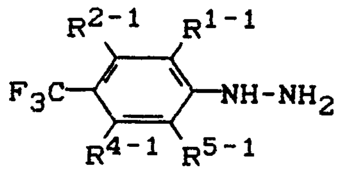

(V)

Tabelle 4

| Bsp. Nr. | | Schmelzpunkt °C |
|---|---|---|
| V -3 | 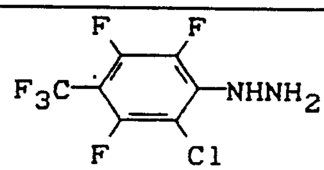 | 93-94 |
| V -4 | 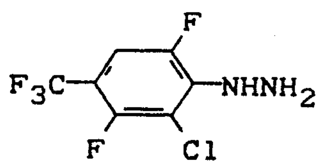 | 72-73 |
| V -5 | 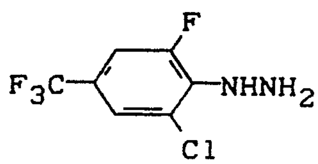 | 60-61 |
| V -6 | 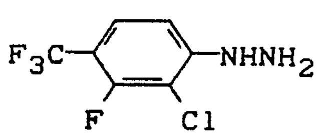 | 102-103 |
| V -7 | 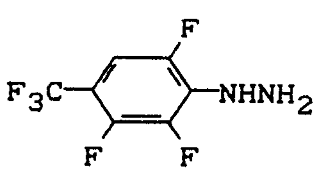 | 60-62 |

Tabelle 4 (Fortsetzung)

| Bsp. Nr. | | Schmelzpunkt °C |
|---|---|---|
| V -8 | 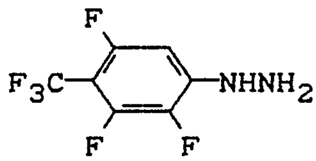 | 69-70 |
| V -9 | 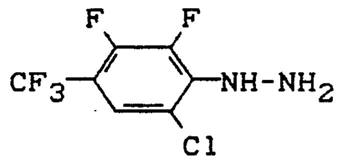 | |

Herstellung der Ausgangsprodukte der Formel (VIII)

Beispiel VIII-1

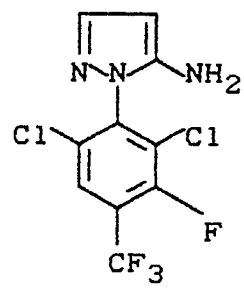

42,6 g (0,162 Mol) 2,6-Dichlor-3-fluor-4-trifluormethylphenylhydrazin werden in 250 ml Methanol p.a. gelöst, mit 35 mg Titriplex III versetzt und zum Rückfluß erhitzt. Bei dieser Temperatur wird innerhalb von 30 Minuten 40 ml (= 44 g/0,049 Mol) 2-Chloracrylnitril 98%ig zugetropft. Es wird 5 Stunden bei Rückflußtemperatur gerührt. Die Reaktionsmischung wird zur Trockne eingeengt. Der Rückstand wird in 100 ml Trifluoressigsäure suspendiert und langsam erwärmt. Es wird 8 Stunden bei 80-83°C gerührt. Die Trifluoressigsäure wird im leichten Vakuum abdestilliert, der Rückstand in 250 ml Methanol gelöst und mit 55 g (0,52 Mol) wasserfreiem Natriumcarbonat versetzt. Es wird 2 Stunden gerührt, das Lösungsmittel im Vakuum abgezogen und der Rückstand in 1 Liter Wasser suspendiert. Der Niederschlag wird abgesaugt, mit Wasser neutral gewaschen und im Vakuum bei 50°C getrocknet.

Man erhält 41,2 g (81 % d.Th.) 5-Amino-1-(2,6-dichlor-3-fluor-4-trifluormethylphenyl)-pyrazol vom Schmelzpunkt 70-78°C.

In entsprechender Weise und gemäß der allgemeinen Angaben zur Herstellung erhält man die folgenden 5-Amino-1-arylpyrazole der allgemeinen Formel (VIII):

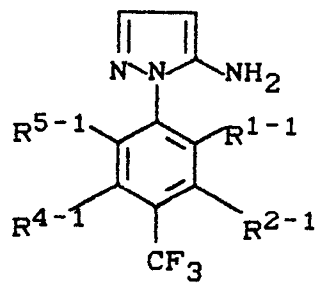

(VIII)

Tabelle 5

| Bsp.-Nr. | 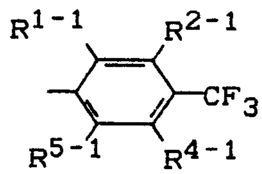 | Schmelzpunkt/° C |
|---|---|---|
| VIII-2 | 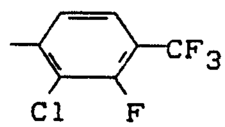 | 112-114 |
| VIII-3 | 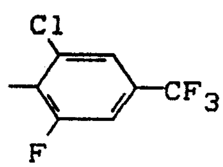 | 81-82 |
| VIII-4 | 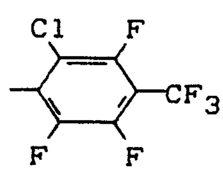 | Öl<br>NMR ($CDCl_3$)<br>$\delta$ = 5.69 ppm (d, 1H),<br>7.55 ppm (d, 1H) |

Tabelle 5 (Fortsetzung)

| Bsp.-Nr. | 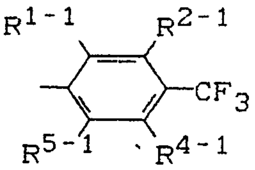 | Schmelzpunkt/° C |
|---|---|---|
| VIII-5 | 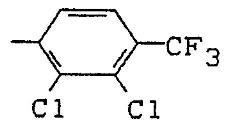 | 139-140 |
| VIII-6 | 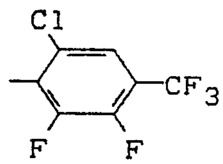 | |
| VIII-7 | 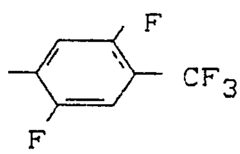 | 95-97 |

## Anwendungsbeispiele

In den folgenden Anwendungsbeispielen wurde die nachstehend aufgeführte Verbindung als Vergleichssubstanz eingesetzt:

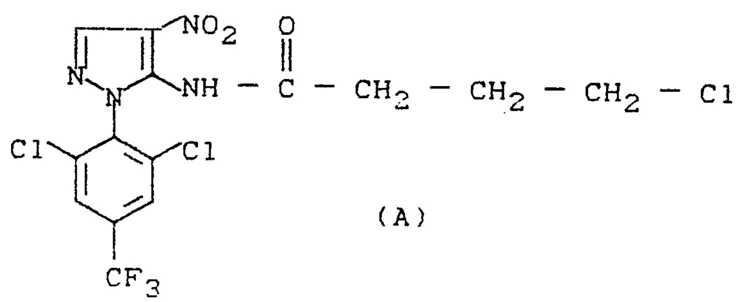

(A)

5-(ω-Chlorbutyradmido)-4-nitro-1-(2,6-dichlor-4-trilfluormethyl-phenyl)-pyrazol (bekannt aus DE-OS 3 402 308).

### Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der herbiziden Wirksamkeit bei Unkräutern wie Amaranthus, Galium, Sinapis, Stellaria und Setaria gegenüber der Vergleichssubstanz (A) zeigen in diesem Test z.B. die Verbindungen gemäß den Herstellungsbeispielen: 1, 2, 3 und 15.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 -15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegen Unkräuter wie Amaranthus, Galium, Matricaria, Sinapis und Panicum bei vergleichbarer Nutzpflanzenselektivität bei z.B. Weizen gegenüber der Vergleichssubstanz (A) zeigen in diesem Test z.B. die Verbindungen gemäß den Herstellungsbeispielen: 2 und 15.

Beispiel C

Entlauben und Austrocknen der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnass mit den Wirkstoffzubereitungen besprüht. Nach 1 Woche werden Blattfall und Austrocknen der Blätter im Vergleich zur Kontrolle boniert. Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

In diesem Test zeigen beispielsweise die Verbindungen gemäß den Herstellungsbeispielen 1, 3, 4, 9 und 15 eine deutliche Wirkung.

**Ansprüche**

1. 5-Dichloracetamido-4-nitro-1-aryl-pyrazole der allgemeinen Formel (I),

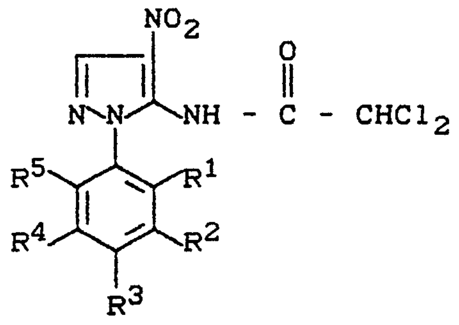

(I)

in welcher
$R^1$ für Wasserstoff, Fluor, Chlor oder Brom steht,
$R^2$ für Wasserstoff, Fluor oder Chlor steht,
$R^3$ für Halogen, Halogenalkyl, Halogenalkoxy, Halogenalkylthio oder Halogenalkylsulfonyl steht,
$R^4$ für Wasserstoff, Fluor oder Chlor steht und
$R^5$ für Fluor, Chlor oder Brom steht,
wobei jedoch
für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht.

2. 5-Dichloracetamido-4-nitro-1-aryl-pyrazole der Formel (I) gemäß Anspruch 1, in welcher
$R^1$ für Wasserstoff, Fluor, Chlor oder Brom steht,
$R^2$ für Wasserstoff, Fluor oder Chlor steht,
$R^3$ für Fluor, Chlor, Brom oder für jeweils geradkettiges oder verzweigtes Halogenalkyl, Halogenalkoxy, Halogenalkylthio oder Halogenalkylsulfonyl mit jeweils 1 bis 4 Kohlenstoffatomen und 1 bis 9 gleichen oder verschiedenen Halogenatomen steht,
$R^4$ für Wasserstoff, Fluor oder Chlor steht und
$R^5$ für Fluor, Chlor oder Brom steht,
wobei jedoch
für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht.

3. Verfahren zur Herstellung von 5-Dichloracetamido-4-nitro-1-aryl-pyrazolen der Formel (I),

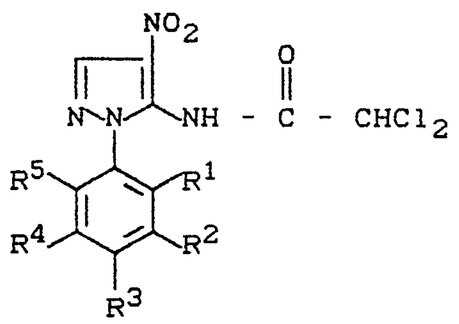

(I)

in welcher
$R^1$ für Wasserstoff, Fluor, Chlor oder Brom steht,
$R^2$ für Wasserstoff, Fluor oder Chlor steht,
$R^3$ für Halogen, Halogenalkyl, Halogenalkoxy, Halogenalkylthio oder Halogenalkylsulfonyl steht,
$R^4$ für Wasserstoff, Fluor oder Chlor steht und
$R^5$ für Fluor, Chlor oder Brom steht,
wobei jedoch

für den Fall, daß $R^1$ und $R^5$ gleichzeitig für Chlor stehen und zusätzlich $R^2$ und $R^4$ gleichzeitig für Wasserstoff stehen, $R^3$ nicht für Chlor oder für Trifluormethyl steht, dadurch gekennzeichnet, daß man

a) 5-Amino-4-nitro-1-aryl-pyrazole der Formel (II),

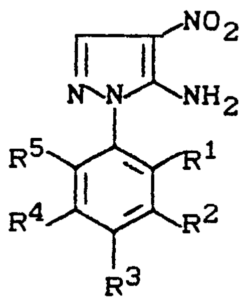

(II)

in welcher
$R^1$, $R^2$, $R^3$, $R^4$ und $R^5$ die oben angegebene Bedeutung haben,
mit Dichloracetylverbindungen der Formel (III),

$Cl_2CH - \overset{O}{\overset{\|}{C}} - E$ (III)

in welcher
E für eine elektronenanziehende Abgangsgruppe steht,
gegebenenfalls in Gegenwart eines Verdünnungsmittels und gegebenenfalls in Gegenwart eines sauren oder basischen Katalysators umsetzt, oder
daß man

b) in 4-Stellung unsubstituierte 5-Dichloracetamido-1-aryl-pyrazole der Formel (IV),

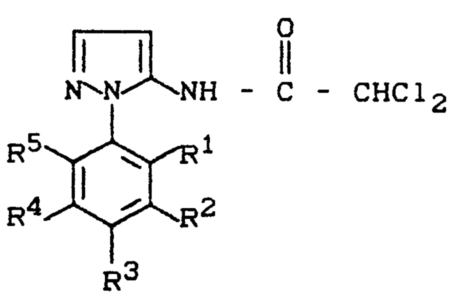

(IV)

in welcher
$R^1$, $R^2$, $R^3$, $R^4$ und $R^5$ die oben angegebene Bedeutung haben,
mit Salpetersäure gegebenenfalls in Gegenwart eines Verdünnungsmittels und gegebenenfalls in Gegenwart eines Katalysators umsetzt.

4. Herbizide und pflanzenwuchsregulierende Mittel, gekennzeichnet durch einen Gehalt an mindestens einem 5-Dichloracetamido-4-nitro-1-aryl-pyrazol der Formel (I) gemäß den Ansprüchen 1 bis 3.

5. Verwendung von 5-Dichloracetamido-4-nitro-1-aryl-pyrazolen der Formel (I) gemäß den Ansprüchen 1 bis 3, zur Bekämpfung von Unkräutern und/oder als Pflanzenwachstumsregulatoren.

6. Verfahren zur Herstellung von herbiziden und/oder pflanzenwuchsregulierenden Mitteln, dadurch gekennzeichnet, daß man 5-Dichloracetamido-4-nitro-1-aryl-pyrazole der Formel (I) gemäß den Ansprüchen 1 bis 3 mit Streckmitteln und/oder oberflächenaktiven Substanzen vermischt.

7. 5-Amino-4-nitropyrazole der Formel (IIa),

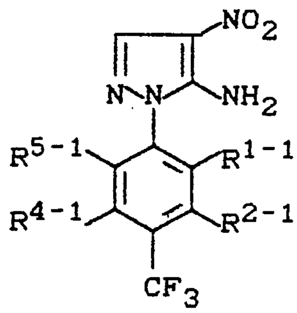

(IIa)

in welcher
$R^{1-1}$, $R^{2-1}$ und $R^{4-1}$ jeweils unabhängig voneinander für Wasserstoff, Fluor oder Chlor stehen und
$R^{5-1}$ für Fluor oder Chlor steht,
wobei jedoch die Kombinationen, in denen
$R^{1-1}$, $R^{2-1}$, $R^{4-1}$ und $R^{5-1}$ gleichzeitig für Fluor stehen, $R^{1-1}$, $R^{2-1}$, $R^{4-1}$ und $R^{5-1}$ gleichzeitig für Chlor stehen, $R^{1-1}$ und $R^{5-1}$ für Chlor und $R^{2-1}$und $R^{4-1}$ entweder gleichzeitig für Wasserstoff oder Fluor stehen, sowie $R^{1-1}$, $R^{2-1}$ und $R^{4-1}$ für Wasserstoff und $R^{5-1}$ gleichzeitig für Chlor stehen und $R^{1-1}$, $R^{2-1}$ und $R^{5-1}$ für Chlor und $R^{4-1}$ gleichzeitig für Wasserstoff stehen, ausgenommen sind.

8. Verfahren zur Herstellung von 5-Amino-4-nitro-pyrazolen der Formel (IIa),

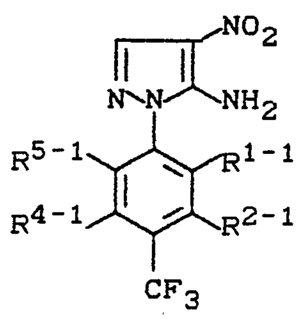

(IIa)

in welcher
$R^{1-1}$, $R^{2-1}$ und $R^{4-1}$ jeweils unabhängig voneinander für Wasserstoff, Fluor oder Chlor stehen und
$R^{5-1}$ für Fluor oder Chlor steht,
wobei jedoch die Kombinationen in denen
$R^{1-1}$, $R^{2-1}$, $R^{4-1}$ und $R^{5-1}$ gleichzeitig für Fluor stehen, $R^{1-1}$, $R^{2-1}$, $R^{4-1}$ und $R^{5-1}$ gleichzeitig für Chlor stehen, $R^{1-1}$ und $R^{5-1}$ für Chlor und $R^{2-1}$und $R^{4-1}$entweder gleichzeitig für Wasserstoff oder Fluor stehen, sowie $R^{1-1}$, $R^{2-1}$ und $R^{4-1}$ für Wasserstoff und $R^{5-1}$ gleichzeitig für Chlor stehen und $R^{1-1}$, $R^{2-1}$ und $R^{5-1}$ für Chlor und $R^{4-1}$ gleichzeitig für Wasserstoff stehen, ausgenommen sind,
dadurch gekennzeichnet, daß man Phenylhydrazine der Formel (V),

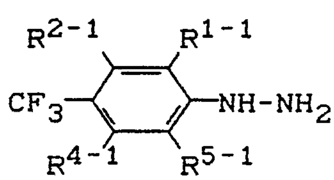

(V)

in welcher
$R^{1-1}$, $R^{2-1}$, $R^{4-1}$ und $R^{5-1}$ die oben angegebene Bedeutung haben,
mit 2-Halogenacrylnitrilen der Formel (VI),

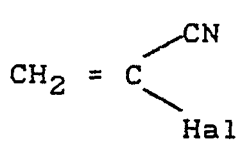

(VI)

in welcher
Hal für Halogen, insbesondere für Chlor oder Brom steht,
zunächst in einer 1. Stufe, gegebenenfalls in Gegenwart eines Verdünnungsmittels, sowie gegebenenfalls in Gegenwart eines Reaktionshilfsmittels bei Temperaturen zwischen -20 °C und +20 °C umsetzt zu den Arylhydrazin-Derivaten der Formel (VII),

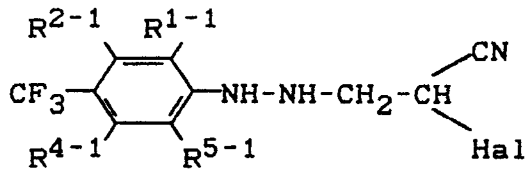
(VII)

in welcher
$R^{1-1}$, $R^{2-1}$, $R^{4-1}$, $R^{5-1}$ und Hal die oben angegebene Bedeutung haben,
und diese in einer 2. Stufe, gegebenenfalls in Gegenwart eines Verdünnungsmittels und gegebenenfalls in Gegenwart eines sauren Katalysators bei Temperaturen zwischen +50 °C und +150 °C cyclisiert,
und die so erhältlichen in 4-Stellung unsubstituierten 5-Amino-pyrazole der Formel (VIII),

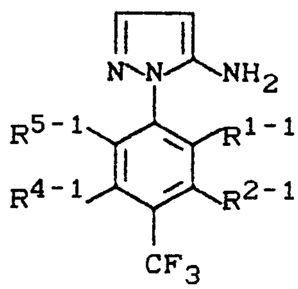
(VIII)

in welcher
$R^{1-1}$, $R^{2-1}$, $R^{4-1}$, $R^{5-1}$ die oben angegebene Bedeutung haben,
in einer Folgereaktion mit einem Nitrierungsmittel gegebenenfalls in Gegenwart eines Verdünnungsmittels und gegebenenfalls in Gegenwart eines Reaktionshilfsmittels bei Temperaturen zwischen -20°C und +50°C nitriert.

9. Arylhydrazine der Formel (V),

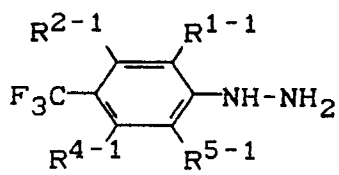
(V)

in welcher
$R^{1-1}$, $R^{2-1}$ und $R^{4-1}$ jeweils unabhängig voneinander für Wasserstoff, Fluor oder Chlor stehen und
$R^{5-1}$ für Fluor oder Chlor steht,
wobei jedoch die Kombination, in denen
$R^{1-1}$, $R^{2-1}$, $R^{4-1}$ und $R^{5-1}$ gleichzeitig für Fluor stehen, $R^{1-1}$, $R^{2-1}$, $R^{4-1}$ und $R^{5-1}$ gleichzeitig für Chlor stehen, $R^{1-1}$ und $R^{5-1}$ für Chlor und $R^{2-1}$und $R^{4-1}$ entweder gleichzeitig für Wasserstoff oder Fluor stehen, sowie $R^{1-1}$, $R^{2-1}$ und $R^{4-1}$ für Wasserstoff und $R^{5-1}$ gleichzeitig für Chlor stehen und $R^{1-1}$, $R^{2-1}$ und $R^{5-1}$ für Chlor und $R^{4-1}$ gleichzeitig für Wasserstoff stehen, ausgenommen sind.

10. Verfahren zur Herstellung von Arylhydrazinen der Formel (V),

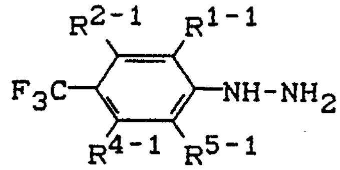

(V)

in welcher

$R^{1-1}$, $R^{2-1}$ und $R^{4-1}$ jeweils unabhängig voneinander für Wasserstoff, Fluor oder Chlor stehen, und

$R^{5-1}$ für Fluor oder Chlor steht,

wobei jedoch die Kombinationen, in denen

$R^{1-1}$, $R^{2-1}$, $R^{4-1}$ und $R^{5-1}$ gleichzeitig für Fluorstehen, $R^{1-1}$, $R^{2-1}$, $R^{4-1}$ und $R^{5-1}$ gleichzeitig für Chlorstehen, $R^{1-1}$ und $R^{5-1}$ für Chlor und $R^{2-1}$und $R^{4-1}$ entweder gleichzeitig für Wasserstoff oder Fluor stehen, sowie $R^{1-1}$, $R^{2-1}$ und $R^{4-1}$ für Wasserstoff und $R^{5-1}$ gleichzeitig für Chlor stehen und $R^{1-1}$, $R^{2-1}$ und $R^{5-1}$ für Chlor und $R^{4-1}$ gleichzeitig für Wasserstoff stehen, ausgenommen sind, und

dadurch gekennzeichnet, daß man Halogenaromaten der Formel (IX)

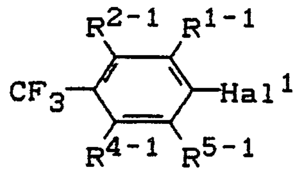

(IX)

in welcher

$R^{1-1}$, $R^{2-1}$, $R^{4-1}$ und $R^{5-1}$ die oben angegebene Bedeutung haben,

$Hal^1$ für Halogen, insbesondere für Fluor oder Chlor steht,

mit Hydrazin oder Hydrazinhydrat gegebenenfalls in Gegenwart eines Verdünnungsmittels umsetzt.